# EUROPEAN PATENT APPLICATION

(11) **EP 4 613 175 A1**
(43) Date of publication of application: **10.09.2025**
(21) Application number: 25160582.0
(22) Date of filing: 27.02.2025
(51) Int. Cl.: A61B 3/10, A61B 5/00, G01N 21/64, G01N 21/76, G02C 5/00, G02C 11/00

(54) **EYEWEAR APPARATUS AND METHOD FOR DETECTING ADMINISTRATION OF EYE DROPS**

(30) Priority: 08.03.2024 FI 20245286
(71) Applicant: Pixieray Oy, 02630 Espoo (FI)
(72) Inventor: Konttila, Antti, 90800 Oulu (FI); Virtanen, Juha, 00560 Helsinki (FI)
(74) Representative: Moosedog Oy

(57) **Abstract**

An eyewear apparatus (100a-c) comprises lens(es) (102a-b); a frame (104); photo emitter(s) (106a-b); photo sensor(s) (108a-b); optical filter(s) (110a-b) arranged to allow light of only a predefined wavelength range to pass therethrough towards the photo sensor(s); and a processor (112). The processor is configured to: collect readings of a photocurrent of the photo sensor(s), whilst the photo emitter(s) is switched on; detect when the readings indicate a presence of a bioluminescence in the predefined wavelength range; and upon said detection, determine whether an eye drop has been administered onto an ocular surface of a user's eye (114), based on at least one of: an intensity of the bioluminescence, a change in the intensity, the eye drop comprising a bioluminescent dye that, when excited by light (116) emitted by the photo emitter(s), emits the bioluminescence of the predefined wavelength range.

## Description

### TECHNICAL FIELD

The present disclosure relates to eyewear apparatuses for detecting administration of eye drops. The present disclosure also relates to methods for detecting administration of eye drops.

### BACKGROUND

Typically, people administer eye drops into their eyes for various reasons such as improving eye comfort, alleviating symptoms, treating medical conditions, maintaining eye health, and the like. However, in some instances, people forget to administer the eye drops into their eyes. Moreover, in some other instances, people administer the eye drops into their eyes, but the eye drops do not reach their eyes. Such instances have led to development of systems for monitoring administration of eye drops.

Presently existing systems for monitoring the administration of eye drops into a person's eyes, are either not sufficiently well-developed or quite expensive. For example, some existing systems are capable of detecting use of eye drop bottles by the person, but they are unable to detect whether the eye drop actually reached the person's eye. Therefore, such existing systems have limited applicability and very limited accuracy of detection of administration of eye drops. Moreover, there exist reusable, stand-alone drug dispensers for administering pills; however, such drug dispensers are not well suited for following up eye drop usage. As another example, some other existing systems employ usage counters that are integrated in single-use drug packages, such as eye drop bottles. However, such usage counters are relatively expensive.

Therefore, in light of the foregoing discussion, there exists a need to overcome the aforementioned drawbacks.

### SUM MARY

The present disclosure seeks to provide an eyewear apparatus and a method for accurately and reliably detecting administration of eye drops in user's eyes. The aim of the present disclosure is achieved by an eyewear apparatus and a method which facilitate a simple, yet accurate and reliable way to detect administration of eye drops, by way of using a bioluminescent dye that, when excited, emits bioluminescence of a predefined wavelength range, as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIGs. 1A, 1B, and 1C illustrate various simplified example implementations of an eyewear apparatus for detecting administration of eye drops, in accordance with various embodiments of the present disclosure;
FIG. 2 illustrates steps of a method for detecting administration of eye drops, in accordance with an embodiment of the present disclosure; and
FIG. 3 illustrates an exemplary graphical representation of a change in an intensity of a bioluminescence as a function of time, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, an embodiment of the present disclosure provides an eyewear apparatus comprising:
at least one lens;
a frame employed to hold the at least one lens;
at least one photo emitter, arranged on at least one of: the frame, a periphery of the at least one lens;
at least one photo sensor, arranged on at least one of: the frame, the periphery of the at least one lens;
at least one optical filter, arranged on an optical path of the at least one photo sensor, to allow light of only a predefined wavelength range to pass therethrough towards the at least one photo sensor; and
a processor configured to:
   collect readings of a photocurrent of the at least one photo sensor, whilst the at least one photo emitter is switched on;
   detect when the readings of the photocurrent indicate a presence of a bioluminescence in the predefined wavelength range; and
   when it is detected that the readings indicate the presence of the bioluminescence in the predefined wavelength range, determine whether an eye drop has been administered onto an ocular surface of a user's eye, based on at least one of: an intensity of the bioluminescence, a change in the intensity of the bioluminescence, wherein the eye drop comprises a bioluminescent dye that, when excited by light emitted by the at least one photo emitter, emits the bioluminescence of the predefined wavelength range.

In a second aspect, an embodiment of the present disclosure provides a method comprising:
collecting readings of a photocurrent of at least one photo sensor of an eyewear apparatus, whilst at least one photo emitter of the eyewear apparatus is switched on, wherein the at least one photo sensor and the at least one photo emitter are arranged on at least one of: a frame, a periphery of at least one lens, of the eyewear apparatus, the frame being employed to hold the at least one lens;
detecting when the readings of the photocurrent indicate a presence of a bioluminescence in a predefined wavelength range, wherein at least one optical filter of the eyewear apparatus allows light of only the predefined wavelength range to pass therethrough towards the at least one photo sensor, the at least one optical filter being arranged on an optical path of the at least one photo sensor; and
when it is detected that the readings indicate the presence of the bioluminescence in the predefined wavelength range, determining whether an eye drop has been administered onto an ocular surface of a user's eye, based on at least one of: an intensity of the bioluminescence, a change in the intensity of the bioluminescence, wherein the eye drop comprises a bioluminescent dye that, when excited by light emitted by the at least one photo emitter, emits the bioluminescence of the predefined wavelength range.

The present disclosure provides the aforementioned eyewear apparatus and the aforementioned method, which facilitate a simple, yet accurate and reliable way to detect administration of the eye drops, by way of using the bioluminescent dye that, when excited, emits the bioluminescence of the predefined wavelength range, in real time or near-real time. Herein, the at least one optical filter allows the light of only the predefined wavelength range to pass therethrough towards the at least one photo sensor and when, upon excitation, the bioluminescent dye emits the bioluminescence, the at least one photo sensor detects the intensity of the bioluminescence and/or the change in the intensity (corresponding to the readings of the photocurrent). Thus, in this way, all elements of the eyewear apparatus synergistically operate in order to determine whether the eye drop has been administered, i.e., whether the eye drop has actually reached the user's eye. Advantageously, the eyewear apparatus and the method facilitate in providing a reliable and efficient confirmation for whether or not the eye drop has been successfully administered onto the ocular surface of the user's eye. The eyewear apparatus is susceptible to be implemented for eye-tracking purposes, in addition to detecting the administration of the eye drops. The eyewear apparatus and the method are simple, robust, fast, support real time detection of administration of the eye drops, and can be implemented with ease.

Throughout the present disclosure, the term *"eyewear apparatus"* refers to an apparatus that is to be worn over user's eyes. Examples of such an eyewear apparatus include, but are not limited to, a pair of glasses, a pair of sunglasses, smart glasses.

Throughout the present disclosure, the term *"lens"* refers to an optical element that is capable of refracting (and optionally, reflecting) light incident thereupon. Optionally, the at least one lens is implemented as a regular lens having a fixed optical power. Alternatively, optionally, the at least one lens is implemented as an adaptive lens whose optical power can be varied. Lenses and optical powers are well-known in the art.

Throughout the present disclosure, the term *"frame"* refers to a structural element that is capable of holding the at least one lens. It will be appreciated that the frame is designed in a manner that the at least one lens is firmly arranged on said frame. In an example, when the eyewear apparatus is implemented as a pair of eyeglasses, the frame may hold two lenses, wherein a first lens is employed for a first eye of the user, and a second lens is employed for a second eye of the user. The first eye could be one of a left eye and a right eye of the user, whereas the second eye could be another of the left eye and the right eye. Optionally, a material of the frame is one of: a plastic, a metal, a polymer, a ceramic.

Throughout the present disclosure, the term *"photo emitter"* refers to an equipment that, in operation, emits the light. Examples of the at least one photo emitter include, but are not limited to, a light-emitting diode (LED), and a laser. The laser may be a vertical-cavity surface-emitting laser (VCSEL), an edge-emitting laser (EEL), or the like. Optionally, the light is infrared (IR) or near-IR light. A technical benefit of employing IR or near-IR light that it is not visible to the user, and therefore, does not cause any disturbance to the user.

The term *"periphery"* refers to an area in a proximity of an edge of the at least one lens. Optionally, said area is on at least one of: a world-facing surface of the at least one lens, a user-facing surface of the at least one lens, a curved surface (on a side) between the world-facing surface and the user-facing surface. It is to be understood that the term *"world-facing surface"* refers to a surface of the at least one lens that faces outward, away from the user's eye, towards a real-world environment in which the user is present. The term *"user-facing surface"* refers to a surface of the at least one lens that faces inward, towards the user's eye. The term *"curved surface"* refers to a narrow, side curved surface that lies between the world-facing surface and the user-facing surface. It will be appreciated that the curved surface is in contact with the frame when the at least one lens is fit inside the frame (for example, having a full rim or a partial rim). Moreover, an area of the curved surface could be a preferrable area for arranging electronic elements (for example such as the at least one photo emitter, the at least one photo sensor, and the at least one optical filter) within the eyewear apparatus. This is because a location of the curved surface enables in concealing said electronic elements in the eyewear apparatus, thereby enhancing its overall aesthetic appeal. In other words, the electronic elements arranged at the curved surface can be discreetly hidden from a view, whilst serving their intended purposes. This facilitates in maintaining a sleek and visually-appealing design of the eyewear apparatus.

Throughout the present disclosure, the term *"photo sensor"* refers to an equipment that is operable to detect (namely, sense) the light incident thereupon. Optionally, the at least one photo sensor is implemented as one of: an IR or near-IR light sensor, a visible light sensor, an ultraviolet (UV) light sensor. Correspondingly, in this regard, the light could be one of: the IR or near-IR light, visible light, UV light. In an example implementation, a given light sensor may be implemented as a photodiode. The photodiode may, for example, be made up of silicon, germanium, indium gallium arsenide, mercury cadmium telluride, and the like. Photodiodes can be employed to detect different types of light, for example, such as the visible light, the IR or near-IR light, and the UV light. Photodiodes are well-known in the art.

Throughout the present disclosure, the term *"optical filter"* refers to an equipment that is capable of allowing the light of the predefined wavelength range to pass therethrough towards the at least one photo sensor, whilst blocking or attenuating the light whose wavelength lies outside said predefined wavelength range. In other words, the at least one optical filter selectively transmits wavelengths of the light lying within the predefined wavelength range, while rejecting other remaining wavelengths of the light. In such a case, the at least one photo sensor would capture the light of only the predefined wavelength range. In some implementations, the predefined wavelength range corresponds to near infrared (NIR) light. In such implementations, the optical filter may be employed to block light of shorter wave lengths than the predefined wavelength range. The optical filter may be any one of: a shortpass near-infrared (NIR) filter, a longpass NIR filter, an interference filter, a dichroic filter.

Notably, the processor controls an overall operation of the eyewear apparatus. For this purpose, the processor is communicably coupled to at least the at least one photo emitter and the at least one photo sensor. It will be appreciated that the at least one processor may comprise a microcontroller or a microprocessor to control operations of the aforesaid components of the eyewear apparatus.

In some implementations, the eyewear apparatus comprises the at least one lens per eye, the at least one photo emitter per eye, the at least one photo sensor per eye, and the at least one optical filter per eye. It will be appreciated that the eye apparatus may comprises a plurality of photo emitters and a plurality of photo sensors, wherein the at least one photo emitter (from amongst the plurality of photo emitters) and the at least one photo sensor (from amongst the plurality of photo sensors) are employed for detecting administration of the eye drops (according to the aforementioned first aspect), while remaining photo emitters and remaining photo sensors may be employed for other purposes, for example, such as eye tracking of the user's eye.

Notably, when the photo emitter is switched on, the readings of the photocurrent are collected by the processor. The photocurrent is an electric current that is generated when the light (emitted by the at least one photo emitter) incident upon the at least one photo sensor (after being transmitted through the at least one optical filter). Since the at least one photo sensor is capable of converting light energy into electrical signals, when photons (namely, particles of the light) strike a photosensitive material of the at least one photo sensor, they typically liberate electrons, thereby creating the electric current. A magnitude of the photocurrent is directly proportional to an intensity of the light incident upon the at least one photo sensor. In other words, greater the intensity (namely, brightness) of the light, greater is the magnitude of the photocurrent, and vice versa. The photocurrent is well-known in the art.

Throughout the present disclosure, the term *"bioluminescence"* refers to a biochemical emission of light. The bioluminescence is emitted in the predefined wavelength range, when the bioluminescent dye present in the eye drop is excited (namely, activated) by the light emitted by the at least one photo emitter. The bioluminescence is well-known in the art.

Throughout the present disclosure, the term *"bioluminescent dye"* refers to a type of dye (namely, a fluorescent compound) that is capable of emitting the bioluminescence (in the predefined wavelength range) when excited by the light emitted by the at least one photo emitter. For example, the bioluminescent dye could be one of: a Fluorescein-based dye, a Rhodol-based dye, a Rhodamine-based dye.

Throughout the present disclosure, the term *"eye drop"* refers to a liquid solution administered onto the ocular surface of the user's eye. Typically, the eye drop is employed for various purposes, for example, such as lubricating dry eyes, administering medication (such as antihistamines, nonsteroidal anti-inflammatory drugs (NSAIDs), antibiotics, antifungals, and the like), rinsing out irritants, or similar. Eye drops are well-known in the art.

It will be appreciated that when the at least one photo sensor does not sense any light in the predefined wavelength range (or may capture negligible/insignificant light) that is reflected off the ocular surface of the user's eye, the readings of the photocurrent may highly likely be zero or near-zero, because the magnitude of the photocurrent is directly proportional to the intensity of the light and the intensity of the bioluminescence (as discussed earlier). Thus, in such a scenario, it is detected the readings of the photocurrent do not indicate the presence of the bioluminescence, and thus it is determined that the eye drop has not been administered onto the ocular surface of the user's eye. It is to be understood that the readings of the photocurrent may also likely be zero or near-zero when the bioluminescent dye present in the eye drop is not excited, for example, when the at least one photo emitter is switched off.

A combination of the at least one photo emitter, the at least one photo sensor, and the at least one optical filter in the eyewear apparatus provides a synergistic effect in accurately detecting the administration of bioluminescent eye drops. This is because the at least one optical filter ensures that only light in the predefined wavelength range corresponding to the bioluminescence is captured by the at least one photo sensor, minimizing interference from ambient light and enhancing eye drop detection reliability. The at least one photo emitter, when activated, excites the bioluminescent dye in the eye drop, ensuring that the emitted bioluminescence is detectable even in low-light conditions or environments with variable lighting. The at least one photo sensor, in turn, measures an intensity of the emitted light with high sensitivity, translating it into a photocurrent whose magnitude directly corresponds to the intensity of the bioluminescence. Such precise interaction between the aforesaid components allows the processor to distinguish between scenarios where the eye drop is actually administered in the user's eye and scenarios where the eye drop is not administered in the user's eye, with no false detections. Furthermore, an integration of the aforesaid components into a single wearable device (namely, the eyewear apparatus) offers real-time, non-invasive monitoring of eye drop administration, ensuring user compliance and improving treatment outcomes.

Furthermore, when the at least one photo sensor senses a significant amount of the light in the predefined wavelength range that is reflected off the ocular surface of the user's eye, the readings of the photocurrent may highly likely be non-zero. This is because the bioluminescent dye present on the ocular surface of the user's eye is excited by the light emitted by the at least one photo emitter; as a result, there would be an abrupt change (namely, a sudden rise) in the magnitude of the photocurrent. Optionally, in this regard, when the readings of the photocurrent exceed a predefined value of the photocurrent, it is detected the readings of the photocurrent indicate the presence of the bioluminescence, and thus it is determined that the eye drop has been administered onto the ocular surface of the user's eye, wherein the predefined value of the photocurrent is a minimum value of the photocurrent above which any value of the photocurrent is indicative of the presence of the bioluminescence. It will be appreciated that the predefined value of the photocurrent of the at least one photo sensor depends on several factors, for example, such as a type of the at least one photo sensor, an amplifier employed for the at least one photo sensor, an intensity of the light emitted by the at least one photo emitter, and similar. Therefore, the predefined value of the photocurrent can be determined experimentally, based on technical specifications of the at least one photo sensor, the at least one photo emitter and other components (for example, such as the amplifier) that are implemented in the eyewear apparatus. Greater the magnitude of the photocurrent, greater is the intensity of the bioluminescence, and vice versa. Similarly, greater the change in the magnitude of the photocurrent with respect to time, greater is the change in the intensity of the bioluminescence with respect to time, and vice versa. It will be appreciated that the processor leverages a non-linear relationship between the magnitude of the photocurrent and the intensity of the bioluminescence to detect abrupt changes indicative of a presence of the bioluminescence. By correlating these changes to the predefined value of the photocurrent determined experimentally based on the technical specifications of the at least one photo emitter, the at least one photo sensor, and additional components such as the amplifier, the eyewear apparatus achieves high reliability and sensitivity in detecting whether the eye drop has been administered. Such a synergistic combination of the aforesaid components not only minimises false positives or negatives but also allows for real-time monitoring and decision-making regarding application of the eye drop. A compact design of the eyewear apparatus ensures that this high-precision detection is achieved without compromising user comfort or usability, making it a viable solution for medical and personal care scenarios.

Optionally, at least a sub-range of a wavelength range of the light emitted by the at least one photo emitter matches an absorption wavelength of the bioluminescent dye. In this regard, the wavelength range of the light emitted by the at least one photo emitter may encompass a spectrum of wavelengths, and the bioluminescent dye may not necessarily be excited by an entirety of the wavelength range of the light. In such a case, at least the sub-range of said wavelength range matches (namely, aligns) with the absorption wavelength of the bioluminescent dye. Due to this, only certain wavelengths of the (emitted) light are absorbed by the bioluminescent dye for triggering an optical activation (namely, excitation) of molecules of the bioluminescent dye, wherein an energy carried by the light (in the sub-range) is transferred to said molecules for subsequently emitting the bioluminescence in the predefined wavelength range. It will be appreciated that by matching at least the sub-range of the wavelength range of the light with the absorption wavelength of the bioluminescent dye, the eyewear apparatus facilitates in achieving an enhanced sensitivity, reliability, and accuracy for detecting the presence of the bioluminescence and subsequently for determining whether or not the eye drop has been administered. Optionally, at least a sub-range of a wavelength range of light that is at least one of: (i) sensed by the at least one photo sensor, (ii) passed through by the at least one optical filter, matches an emission wavelength of the bioluminescent dye. This ensures that the at least one photo sensor detects only the emitted bioluminescence while minimising interference from other wavelengths, such as ambient light or reflected light outside the predefined wavelength range. Consequently, this feature further enhances reliability and robustness of the eyewear apparatus in detecting the presence of the bioluminescence and determining whether or not the eye drop has been administered, particularly in diverse environmental lighting conditions.

Optionally, the predefined wavelength range corresponds to near infrared (NIR) light. In this regard, preferably, the bioluminescent dye may be selected in a manner that the predefined wavelength range of the emission of the bioluminescence corresponds to an NIR region. The technical benefit of the predefined wavelength range to correspond to the NIR light (which typically falls outside a visible spectrum) is that the bioluminescence is invisible (namely, unnoticeable) to the user's eyes. Thus, in this way, the presence of the bioluminescence and administration of the eye drops could be highly accurately and reliably detected, whilst a user of the eyewear apparatus experiences minimal visual interference or distraction, allowing for a comfortable and uninterrupted usage of the eyewear apparatus. In other words, usage of the NIR light enhances comfort and usability of the eyewear apparatus by ensuring that the user is not distracted by visible light, whilst still achieving highly accurate and reliable detection of the bioluminescence and the administration of the eye drops. Typically, a wavelength of the NIR light lies in a range of 700 nanometres (nm) to 2500 nm.

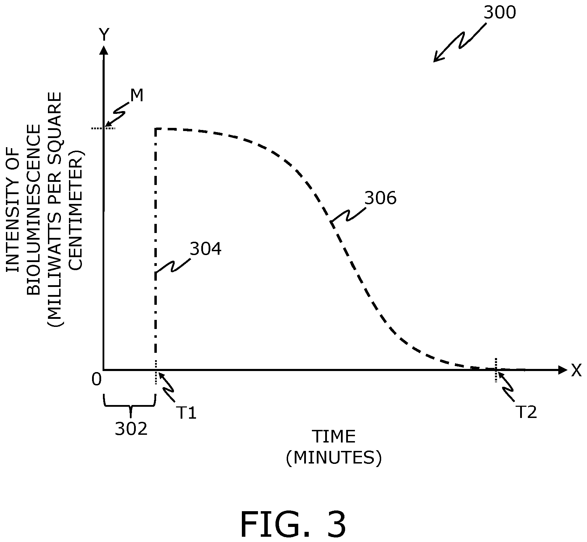

Optionally, the processor is configured to calculate the change in the intensity of the bioluminescence over a predefined time period. In this regard, at a time instant when the eye drop comprising the bioluminescent dye is administered onto the ocular surface and the bioluminescent dye is excited by the light emitted by the at least one photo emitter, the intensity of the bioluminescence increases drastically with respect to time (namely, a rate of the change in the intensity of the bioluminescence is high and the magnitude of the photocurrent would be high, because there is a maximum amount of the eye drop on the ocular surface just after administration), and reaches a maximum value. As the amount of the eye drop on the ocular surface gradually diminishes, for example, due to absorption into tissues of the user's eye, eye blinking, tear production, and the like, the intensity of the bioluminescence also gradually decreases with respect to time (namely, a rate of the change in the intensity is gradually decreasing as the magnitude of the photocurrent would also be decreasing gradually), and reaches a zero or near-zero value. Therefore, in this regard, the change in the intensity of the bioluminescence over the predefined time period corresponds to a decline in the intensity of the bioluminescence (i.e., the change could be represented by a declining curve as a function of time). Such a declining curve has been also illustrated in conjunction with FIG. 3, for sake of clarity and better understanding. It is to be understood that prior to administration of the eye drop, the intensity of the bioluminescence may be zero or near-zero. Optimally, the predefined time period lies in a range of 5 seconds to 10 minutes; more optimally, in a range of 5 seconds to 5 minutes; yet more optimally, in a range of 5 seconds to 1 minute. By calculating the change in the intensity over the predefined time period, the processor enhances a precision of detecting a bioluminescence decay, allowing for a more accurate determination of how much of the eye drop remains on the ocular surface over the predefined time period. This provides a more nuanced and reliable assessment for detecting administration of the eye drop. An ability to track the change in the intensity over administration of the eye drop may also allow for better temporal calibration, ensuring that any fluctuations in the intensity due to factors such as eye blinking or tear production are accounted for, leading to accurate results in bioluminescence detection, for detecting administration of the eye drop.

Optionally, the at least one lens comprises a plurality of nanoparticles that are employed to direct a portion of the light incident thereupon towards the at least one photo sensor. In this regard, the plurality of nanoparticles within the at least one lens facilitate in redirecting an optical path of the portion of the (incident) light towards the at least one photo sensor. Beneficially, due to this, the at least one photo sensor is able to capture a significant amount of the (bioluminescence) light (because of an improved signal-to-noise ratio), and generates the photocurrent accordingly. Thus, in this way, the presence of the bioluminescence and administration of the eye drops could be highly accurately and reliably detected. Optionally, the plurality of nanoparticles are dispersed in a substrate of the at least one lens. Alternatively, optionally, the plurality of nanoparticles are dispersed in a coating formed on a substrate of the at least one lens. In this regard, any well-known coating deposition technique (such as a spin coating technique, a dip coating technique, a spray coating technique, or similar) could be utilised for forming said coating. It will be appreciated that a given nanoparticle could be one of: a gold nanoparticle, a silver nanoparticle, a silica nanoparticle, a titania nanoparticle.

Moreover, optionally, the processor is configured to:
collect at least one baseline reading of the photocurrent of the at least one photo sensor, prior to detection of the readings indicating the presence of the bioluminescence in the predefined wavelength range; and
determine a baseline intensity of the bioluminescence, based on the at least one baseline reading of the photocurrent.

In this regard, prior to an administration of the eye drop, there may be a scenario wherein the at least one photo sensor may not likely detect any presence of the bioluminescence, and thus the baseline intensity of the bioluminescence would be zero or near-zero (as the at least one baseline reading of the photocurrent is zero or near-zero). However, there may be another scenario wherein a real-world environment in which the user of the eyewear apparatus is present comprises a light source, wherein the light source emits light in a wavelength range that at least partially matches with the predefined wavelength range of the bioluminescence. In such a scenario, it may be highly likely that the at least one photo sensor detects said light and generates the photocurrent accordingly (i.e., the photocurrent is non-zero in such a case). Thus, said (non-zero) photocurrent would be considered as the at least one baseline reading of the photocurrent, as said photocurrent is not actually generated due to the presence of the bioluminescence, but is rather generated due to presence of said light whose wavelength range at least partially matches with the predefined wavelength range. Thus, in the aforesaid scenario, an intensity of the light that is detected by the at least one photo sensor in an absence of the eye drop on the ocular surface can be considered as the baseline intensity of the bioluminescence. The term *"baseline reading"* of the photocurrent refers to a reading of the photocurrent that is collected prior to the administration of the eye drop. The term *"baseline intensity"* of the bioluminescence refers to an intensity of the bioluminescence that is detected prior to the administration of the eye drop. It will be appreciated that the baseline intensity serves as a reference point when subsequent readings of the photocurrent are collected after the eye drop has been administered. Thus, any contribution due to the aforesaid light source could be effectively subtracted from an initial value of the intensity of the bioluminescence to determine a correct (namely, calibrated or adjusted) value of the intensity of the bioluminescence (as discussed later in detail). In other words, the baseline intensity facilitates in improving an accuracy and reliability of detecting the presence of the bioluminescence. The light source could be a natural light source (such as the Sun) or an artificial light source (such as a lamp). It will be appreciated that by accounting for the baseline intensity of the bioluminescence, the eyewear apparatus may be susceptible to accurately distinguish between the bioluminescence from the eye drop and ambient light, resulting in a reliable and consistent determination of eye drop administration, regardless of any ambient light conditions. Such an approach may enable a dynamic calibration of the eyewear apparatus in real-time, ensuring that variations in the ambient light do not skew a detection of bioluminescence, making the eyewear apparatus precise in varying usage conditions.

Optionally, the at least one baseline reading comprises lighted baseline reading and non-lighted baseline reading corresponding to when the at least one photo emitter is switched on and when the at least one photo emitter is switched off, respectively, wherein the baseline intensity of the bioluminescence is determined, based on a difference in the lighted baseline reading and the non-lighted baseline reading. Herein the term *"lighted baseline reading"* of the photocurrent refers to a reading of the photocurrent that is collected prior to the administration of the eye drop when the at least one photo emitter is switched on. The term *"non-lighted baseline reading"* of the photocurrent refers to a reading of the photocurrent that is collected prior to the administration of the eye drop when the at least one photo emitter is switched off. It will be appreciated that when the at least one photo emitter is switched on and when the at least one photo emitter switched off, the at least one photo sensor may detect different intensities of light whose wavelength range at least partially matches with the predefined wavelength range, wherein said light comprises an ambient light in the real-world environment when the at least one photo emitter is switched off, and comprises the ambient light and the light emitted by the at least one photo emitter when the at least one photo emitter is switched on. Optionally, in this regard, the processor is configured to determine the baseline intensity as a difference between an intensity of the light corresponding to the lighted baseline reading and an intensity of the light corresponding to the non-lighted baseline reading. Advantageously, the baseline intensity determined in the aforesaid manner facilitates in improving an accuracy and reliability of detecting the presence of the bioluminescence and the administration of the eye drops. This may be because by taking into account both the lighted baseline reading and the non-lighted baseline reading, the processor would be able to differentiate between any ambient light and light emitted by the at least one photo emitter. This ensures that the ambient light does not interfere with readings that are used to detect the presence of the bioluminescence. Since the non-lighted baseline reading accounts for the ambient light, the eyewear apparatus can effectively neutralise any potential variations in environmental lighting. By subtracting the ambient light from a total detected light when the at least one phot emitter is active, the processor ensures that only bioluminescence contribution is considered, further reducing a likelihood of false positives caused by external lighting conditions and improving an accuracy and reliability of detecting the administration of the eye drops.

Optionally, the processor is configured to:
determine an initial value of the intensity of the bioluminescence from the readings indicating the presence of the bioluminescence; and
calculate a correct value of the intensity of the bioluminescence by subtracting the baseline intensity of the bioluminescence from the initial value of the intensity of the bioluminescence.

Optionally, in this regard, when determining the initial value of the intensity of the bioluminescence, the processor is configured to utilise a predetermined correlation between the readings of the photocurrent and corresponding intensities of the bioluminescence. Such a predetermined correlation can be obtained from a calibration process. It will be appreciated that since the initial value of the intensity of the bioluminescence may also include the baseline intensity of the bioluminescence (that is undesirable and irrelevant for determining the administration of the eye drops), the processor calculates the correct value of the intensity of the bioluminescence by performing the aforesaid subtraction. Beneficially, the correct value of the intensity of the bioluminescence determined in the aforesaid manner facilitates in improving an accuracy and reliability of detecting the administration of the eye drops.

The present disclosure also relates to the method as described above. Various embodiments and variants disclosed above, with respect to the aforementioned first aspect, apply *mutatis mutandis* to the method.

Optionally, the method further comprises calculating the change in the intensity of the bioluminescence over a predefined time period.

Optionally, the method further comprises:
collecting at least one baseline reading of the photocurrent of the at least one photo sensor, prior to performing the step of detecting the readings indicating the presence of the bioluminescence in the predefined wavelength range; and
determining a baseline intensity of the bioluminescence, based on the at least one baseline reading of the photocurrent.

Optionally, in the method, the at least one baseline reading comprises lighted baseline reading and non-lighted baseline reading corresponding to when the at least one photo emitter is switched on and when the at least one photo emitter is switched off, respectively, wherein the baseline intensity of the bioluminescence is determined, based on a difference in the lighted baseline reading and the non-lighted baseline reading.

Optionally, the method further comprises:
determining an initial value of the intensity of the bioluminescence from the readings indicating the presence of the bioluminescence; and
calculating a correct value of the intensity of the bioluminescence by subtracting the baseline intensity of the bioluminescence from the initial value of the intensity of the bioluminescence.

Optionally, in the method, at least a sub-range of a wavelength range of the light emitted by the at least one photo emitter matches an absorption wavelength of the bioluminescent dye.

Optionally, in the method, the at least one lens comprises a plurality of nanoparticles that are employed to direct a portion of the light incident thereupon towards the at least one photo sensor.

Optionally, in the method, the predefined wavelength range corresponds to near infrared (NIR) light.

### DETAILED DESCRIPTION OF THE DRAWINGS

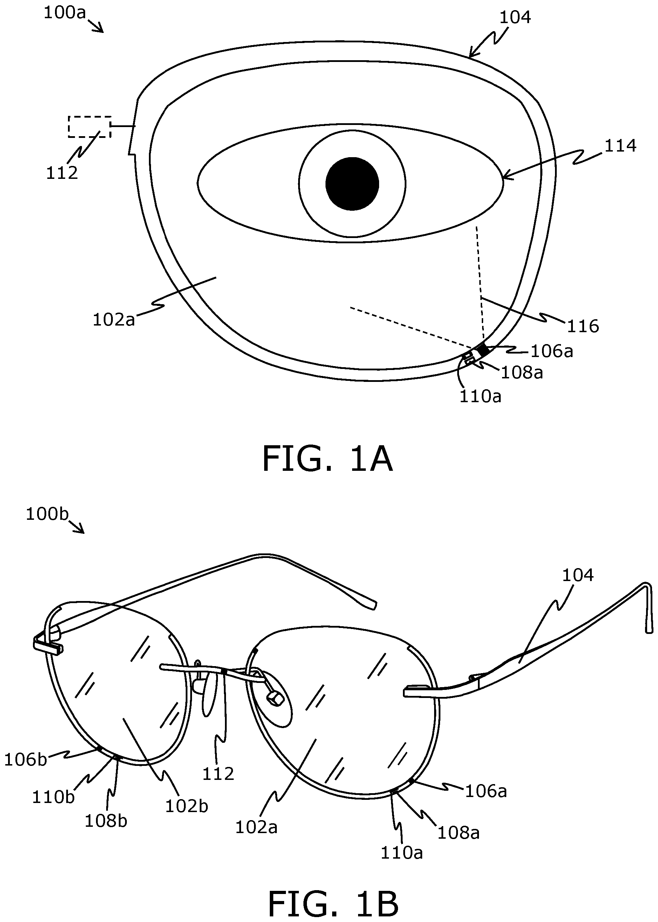

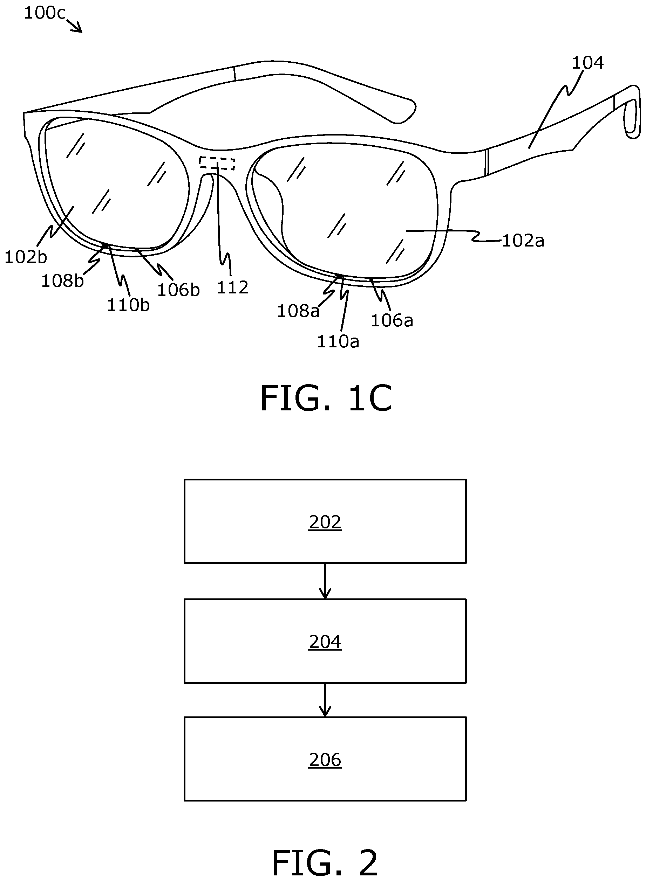

Referring to FIGs. 1A, 1B, and 1C, illustrated are various simplified example implementations of an eyewear apparatus **100a, 100b,** and **100c** for detecting administration of eye drops, in accordance with various embodiments of the present disclosure.

With reference to FIG. 1A, the eyewear apparatus **100a** comprises at least one lens (depicted as a lens **102a),** a frame **104,** at least one photo emitter (depicted as a photo emitter **106a),** at least one photo sensor (depicted as a photo sensor **108a),** at least one optical filter (depicted as an optical filter **110a),** and a processor **112.** The processor **112** is communicably coupled to the photo emitter **106a** and the photo sensor **108a.** The frame **104** is employed to hold the lens **102a.** For sake of simplicity and convenience, only a portion of the frame **104** is shown. The photo emitter **106a** and the photo sensor **108a** are shown to be arranged on (a periphery of) the frame **104.** The optical filter **110a** is shown to be arranged on (the periphery of) the frame **104,** along an optical path of the photo sensor **108a.**

For sake of simplicity and convenience, only a portion of the eyewear apparatus **100a** that corresponds to a given eye **114** of a user is shown in FIG. 1A. The given eye **114** could be any one of: a left eye of the user, a right eye of the user. The photo emitter **106a** is shown to emit light **116** (depicted using dashed lines) towards the given eye **114.**

With reference to FIGs. 1B and 1C, each of the eyewear apparatuses **100b** and **100c** comprises at least one lens per eye (depicted as a lens **102a** for the left eye, and another lens **102b** for the right eye), a frame **104,** at least one photo emitter (depicted as a photo emitter **106a** for the left eye, and another photo emitter **106b** for the right eye), at least one photo sensor (depicted as a photo sensor **108a** for the left eye, and another photo sensor **108b** for the right eye), at least one optical filter (depicted as an optical filter **110a** for the left eye, and another optical filter **110b** for the right eye), and a processor **112.** The processor **112** is communicably coupled to the photo emitters **106a-b** and the photo sensors **108a-b.** The frame **104** is employed to hold the lenses **102-b.** The photo emitters **106a-b,** in operation, emit light (for example, the light **116)** towards a given eye (for example, the given eye **114).** The optical filters **110a-b** are shown to be arranged along respective optical paths of the photo sensors **108a-b.**

With reference to FIG. 1B, the photo emitters **106a-b** and the photo sensors **108a-b** are shown to be arranged on areas of user-facing surfaces of the lenses **102a-b,** respectively. The frame **104** has been shown as a rimless frame. With reference to FIG. 1C, the photo emitters **106a-b** and the photo sensors **108a-b** are shown to be arranged on areas of curved surfaces (on sides) between user-facing surfaces and world-facing surfaces of the lenses **102a-b,** respectively. Said curved surfaces are in contact with the frame **104** when the lenses **102a-b** are fit inside the frame **104.** The frame **104** has been shown as a full-rim frame.

It may be understood by a person skilled in the art that FIGs. 1A-1C include simplified example implementations of the eyewear apparatuses **100a-c,** for sake of clarity, which should not unduly limit the scope of the claims herein. It is to be understood that specific implementations of the eyewear apparatuses **100a-c** are provided as an example and are not to be construed as limiting them to specific numbers or types of lenses, frames, photo emitters, photo sensors, optical filters, and processors. The person skilled in the art will recognize many variations, alternatives, and modifications of embodiments of the present disclosure.

Referring to FIG. 2, illustrated are steps of a method for detecting administration of eye drops, in accordance with an embodiment of the present disclosure. At step **202,** readings of a photocurrent of at least one photo sensor of the eyewear apparatus are collected, whilst at least one photo emitter of the eyewear apparatus is switched on, wherein the at least one photo sensor and the at least one photo emitter are arranged on at least one of: a frame, a periphery of at least one lens, of the eyewear apparatus, the frame being employed to hold the at least one lens. At step **204,** it is detected when the readings of the photocurrent indicate a presence of a bioluminescence in a predefined wavelength range, wherein at least one optical filter of the eyewear apparatus allows light of only the predefined wavelength range to pass therethrough towards the at least one photo sensor, the at least one optical filter being arranged on an optical path of the at least one photo sensor. When it is detected that the readings indicate the presence of the bioluminescence in the predefined wavelength range, at step **206,** it is determined whether an eye drop has been administered onto an ocular surface of a user's eye, based on at least one of: an intensity of the bioluminescence, a change in the intensity of the bioluminescence, wherein the eye drop comprises a bioluminescent dye that, when excited by light emitted by the at least one photo emitter, emits the bioluminescence of the predefined wavelength range.

The aforementioned steps are only illustrative and other alternatives can also be provided where one or more steps are added, one or more steps are removed, or one or more steps are provided in a different sequence without departing from the scope of the claims.

Referring to FIG. 3, illustrated is an exemplary graphical representation **300** of a change in an intensity of a bioluminescence as a function of time, in accordance with an embodiment of the present disclosure. With reference to FIG. 3, a positive X-axis represents time (for example, in minutes), and a positive Y-axis represents the intensity of the bioluminescence (for example, in milliwatts per square centimetre). A portion **302** in the graphical representation **300** corresponds to a time period between 0 (i.e., zero) and a time instant **T1** during which an eye drop (comprising a bioluminescent dye) has not been administered onto an ocular surface of a user's eye. As shown, within said time period, the intensity of the bioluminescence is zero or nearly zero. A portion **304** (depicted using a dash-dot line) in the graphical representation **300** corresponds to the time instant **T1** when said eye drop is administered onto the ocular surface of the user's eye. As shown, at the time instant **T1,** the intensity of the bioluminescence increases steeply with respect to time, until it reaches a maximum intensity **M** of the bioluminescence. This happens because the bioluminescent dye in the eye drop is excited by light emitted by at least one photo emitter of an eyewear apparatus, thereby emitting the bioluminescence of a predefined wavelength range. A portion **306** (depicted using a dashed-line curve that is similar to a sigmoid curve) in the graphical representation **300** corresponds to a time period between the time instant **T1** and a time instant **T2** during which the intensity of the bioluminescence decreases gradually with respect to time, from the maximum intensity **M** of the bioluminescence to a minimum intensity of the bioluminescence, i.e., zero or nearly zero. This happens because an amount of the eye drop (comprising the bioluminescent dye) gradually diminishes on the ocular surface of the user's eye, for example, due to absorption into tissues of the user's eye, eye blinking, tear production, and the like. The time period between the time instant **T1** and the time instant **T2** may, for example, lie in a range from 1 minute to 10 minutes.

FIG. 3 is merely an example, which should not unduly limit the scope of the claims herein. The person skilled in the art will recognize many variations, alternatives, and modifications of embodiments of the present disclosure.

## Claims

1. An eyewear apparatus (100a-c) comprising:
at least one lens (102a-b);
a frame (104) employed to hold the at least one lens;
at least one photo emitter (106a-b), arranged on at least one of: the frame, a periphery of the at least one lens;
at least one photo sensor (108a-b), arranged on at least one of: the frame, the periphery of the at least one lens;
at least one optical filter (110a-b), arranged on an optical path of the at least one photo sensor, to allow light of only a predefined wavelength range to pass therethrough towards the at least one photo sensor; and
a processor (112) configured to:
collect readings of a photocurrent of the at least one photo sensor, whilst the at least one photo emitter is switched on;
detect when the readings of the photocurrent indicate a presence of a bioluminescence in the predefined wavelength range; and
when it is detected that the readings indicate the presence of the bioluminescence in the predefined wavelength range, determine whether an eye drop has been administered onto an ocular surface of a user's eye (114), based on at least one of: an intensity of the bioluminescence, a change in the intensity of the bioluminescence, wherein the eye drop comprises a bioluminescent dye that, when excited by light (116) emitted by the at least one photo emitter, emits the bioluminescence of the predefined wavelength range.

2. The eyewear apparatus (100a-c) of claim 1, wherein the processor (112) is configured to calculate the change in the intensity of the bioluminescence over a predefined time period.

3. The eyewear apparatus (100a-c) of any of the preceding claims, wherein the processor (112) is configured to:
collect at least one baseline reading of the photocurrent of the at least one photo sensor (108a-b), prior to detection of the readings indicating the presence of the bioluminescence in the predefined wavelength range; and
determine a baseline intensity of the bioluminescence, based on the at least one baseline reading of the photocurrent.

4. The eyewear apparatus (100a-c) of claim 3, wherein the at least one baseline reading comprises lighted baseline reading and non-lighted baseline reading corresponding to when the at least one photo emitter (106a-b) is switched on and when the at least one photo emitter is switched off, respectively, wherein the baseline intensity of the bioluminescence is determined, based on a difference in the lighted baseline reading and the non-lighted baseline reading.

5. The eyewear apparatus (100a-c) of claim 3 or 4, wherein the processor (112) is configured to:
determine an initial value of the intensity of the bioluminescence from the readings indicating the presence of the bioluminescence; and
calculate a correct value of the intensity of the bioluminescence by subtracting the baseline intensity of the bioluminescence from the initial value of the intensity of the bioluminescence.

6. The eyewear apparatus (100a-c) of any of the preceding claims, wherein at least a sub-range of a wavelength range of the light (116) emitted by the at least one photo emitter (106a-b) matches an absorption wavelength of the bioluminescent dye.

7. The eyewear apparatus (100a-c) of any of the preceding claims, wherein the at least one lens (102a-b) comprises a plurality of nanoparticles that are employed to direct a portion of the light (116) incident thereupon towards the at least one photo sensor (108a-b).

8. The eyewear apparatus (100a-c) of any of the preceding claims, wherein the predefined wavelength range corresponds to near infrared (NIR) light.

9. A method comprising:
collecting readings of a photocurrent of at least one photo sensor (108a-b) of an eyewear apparatus (100a-c), whilst at least one photo emitter (106a-b) of the eyewear apparatus is switched on, wherein the at least one photo sensor and the at least one photo emitter are arranged on at least one of: a frame (104), a periphery of at least one lens (102a-b), of the eyewear apparatus, the frame being employed to hold the at least one lens;
detecting when the readings of the photocurrent indicate a presence of a bioluminescence in a predefined wavelength range, wherein at least one optical filter (110a-b) of the eyewear apparatus allows light of only the predefined wavelength range to pass therethrough towards the at least one photo sensor, the at least one optical filter being arranged on an optical path of the at least one photo sensor; and
when it is detected that the readings indicate the presence of the bioluminescence in the predefined wavelength range, determining whether an eye drop has been administered onto an ocular surface of a user's eye (114), based on at least one of: an intensity of the bioluminescence, a change in the intensity of the bioluminescence, wherein the eye drop comprises a bioluminescent dye that, when excited by light (116) emitted by the at least one photo emitter, emits the bioluminescence of the predefined wavelength range.

10. The method of claim 9, further comprising calculating the change in the intensity of the bioluminescence over a predefined time period.

11. The method of claim 9 or 10, further comprising:
collecting at least one baseline reading of the photocurrent of the at least one photo sensor (108a-b), prior to performing the step of detecting the readings indicating the presence of the bioluminescence in the predefined wavelength range; and
determining a baseline intensity of the bioluminescence, based on the at least one baseline reading of the photocurrent.

12. The method of claim 11, wherein the at least one baseline reading comprises lighted baseline reading and non-lighted baseline reading corresponding to when the at least one photo emitter (106a-b) is switched on and when the at least one photo emitter is switched off, respectively, wherein the baseline intensity of the bioluminescence is determined, based on a difference in the lighted baseline reading and the non-lighted baseline reading.

13. The method of claim 11 or 12, further comprising:
determining an initial value of the intensity of the bioluminescence from the readings indicating the presence of the bioluminescence; and
calculating a correct value of the intensity of the bioluminescence by subtracting the baseline intensity of the bioluminescence from the initial value of the intensity of the bioluminescence.

14. The method of any of claims 9-13, wherein at least a sub-range of a wavelength range of the light (116) emitted by the at least one photo emitter (106a-b) matches an absorption wavelength of the bioluminescent dye.

15. The method of any of claims 9-14, wherein the at least one lens (102a-b) comprises a plurality of nanoparticles that are employed to direct a portion of the light (116) incident thereupon towards the at least one photo sensor (108a-b).
